# EUROPÄISCHE PATENTSCHRIFT

(11) **EP 1 752 138 B1**
(45) Veröffentlichungstag und Bekanntmachung des Hinweises auf die Patenterteilung: **17.11.2010**
(21) Anmeldenummer: 06014558.8
(22) Anmeldetag: 13.07.2006
(51) Int. Cl.: A61K 8/92, A61Q 5/02, A61Q 5/06, A61Q 5/08, A61Q 5/10, A61Q 5/12

(54) **Amaranthsamenöl in Haarbehandlungsmitteln**
Amaranth seed oil in hair compositions
Huile de graine d'amarante dans les compositions capillaires

(30) Priorität: 11.08.2005 DE 102005038359
(43) Veröffentlichungstag der Anmeldung: 14.02.2007
(73) Patentinhaber: Henkel AG & Co. KGaA, 40589 Düsseldorf (DE)
(72) Erfinder: Schulze zur Wiesche, Erik, 20251 Hamburg (DE); Banowski, Bernhard, 40597 Düsseldorf (DE); Höffkes, Horst, 40595 Düsseldorf (DE); Somfleth, Petra, 21629 Neu Wulmstorf (DE); Brockmann, Claudia, 40627 Düsseldorf (DE)

(56) Entgegenhaltungen:
- FR-A1- 2 746 303
- DATABASE EPODOC EUROPEAN PATENT OFFICE, THE HAGUE, NL; XP002413271 -& KR 2004 111 227 A (ABLE C & C CO LTD) 31. Dezember 2004 (2004-12-31)

## Beschreibung

Die Erfindung betrifft kosmetische Mittel enthaltend Amaranthsamenöl sowie die Verwendung zur Behandlung keratinischer Fasern, insbesondere zur Farbveränderung, Reinigung und/oder Pflege von Haaren.

Durch die vielfältige Behandlung der Haare, beispielsweise durch Bleichung, Färbung, Tönung und Verformung, aber auch durch die Reinigung der Haare mit aggressiven Shampoos und durch Umweltbelastungen, kann es zu einer unerwünschten Beeinträchtigung der Haarstruktur kommen. Die Beeinträchtigung der Haarstruktur macht sich beispielsweise in einer schlechten Nass- und Trockenkämmbarkeit, einer verstärkten elektrostatischen Aufladung, verstärkter Sprödigkeit, verringerter Höchstreißkraft und Reißdehnung der Haare, Spliss und einem insgesamt verschlechterten äußeren Erscheinungsbild der Haare bemerkbar. Des weiteren besteht bei einer Vielzahl kosmetischer Haarpflegestoffe das Problem, dass sie in Testformulierungen herausragende Ergebnisse hinsichtlich Haarpflege und -konditionierung liefern, in Shampoo- und/oder Konditionierformulierungen jedoch an Effizienz verlieren, weil sie sich nicht oder nicht in ausreichendem Maße in diese Formulierungen einarbeiten lassen.

Es sind zahlreiche Wirkstoffe bekannt, die die Haarpflege unterstützen und Beeinträchtigungen der Haarstruktur vorbeugen. Die bekannten Wirkstoffe können jedoch nicht alle Bedürfnisse in ausreichendem Maße abdecken.

Es besteht daher der Bedarf nach Wirkstoffen bzw. Wirkstoffkombinationen für kosmetische Mittel mit guten pflegenden Eigenschaften und guter biologischer Abbaubarkeit, die sich problemlos in bekannte Formulierungen einarbeiten lassen.

Weiterhin besteht der Bedarf nach Haarbehandlungsmitteln, die das Haar reinigen und pflegen, ohne dass zwingend ein weiterer Haarbehandlungsschritt mit einem speziellen Haarpflegemittel notwendig ist. Aufgrund der steigenden Absatzzahl von Haarfärbemitteln, die die Haarstruktur insbesondere bei wiederholter Haarfärbung oder Haarbleichung immer mehr schädigen, werden gleichzeitig Haarbehandlungsmittel angestrebt, die eine durch oxidative Behandlung bedingte Haarschädigung verringern und das Haar in einen guten Zustand hinsichtlich Nass- und Trockenkämmbarkeit, Glanz, Weichheit, Volumen, Griff und Halt versetzten. Gleichzeitig soll Spliss verringert bzw. dem Haarspliss vorgebeugt werden.

Natürliche Ölkomponenten werden seit langem vielfach in kosmetischen Mitteln eingesetzt. Hierzu sei auf die einschlägige Fachliteratur verwiesen, beispielsweise in K. Schrader, "Grundlagen und Rezepturen der Kosmetika", 2. Auflage, Seite 329 bis 341, Hüthig Buch Verlag Heidelberg.

Es ist weiterhin bekannt, zur kosmetischen Hautbehandlung, insbesondere von Schuppenflechte und Neurodermitis, Amaranthsamenöl alleine oder als Gemisch einzusetzen (EP 875 156 B1 und JP 11 060 442 A).

WO97/34579 A1 betrifft einen festen, pulverförmigen Amaranthsamenextrakt, der durch Extraktion von Amaranthsamen mit einem hydrophilen Lösungsmittel wie Wasser, Alkohol und/oder Aceton, und anschließendes Abdampfen des Lösungsmittels gewonnen wird, und der sich als Pflegekomponente in Haarbehandlungsmitteln eignet und den Haaren mehr Weichheit, eine bessere Entwirrung, mehr Volumen und eine verbesserte Hydratation der Kopfhaut verleiht. Bislang nicht bekannt ist jedoch die Verwendung von Amaranthsamenöl in Haarbehandlungsmitteln, das durch Extraktion oder Pressverfahren der Amaranthsamen gewonnen wird.

Es wurde nun überraschenderweise gefunden, dass sich Amaranthsamenöl problemlos in Haarreinigungs- und, -pflegemittel einarbeiten lässt, und den Haaren mehr Glanz sowie verbesserte Pflegeeigenschaften, insbesondere verbesserte Kämmeigenschaften, mehr Glanz und einen verbesserten Griff, verleiht Insbesondere wurde gefunden, dass ein Haarreinigungsmittel auf der Basis des erfindungsgemäßen Amarantsamenöls mit geringsten Mengen an Lösungsvermittler auskommt, während identische Zusammenstzungen auf der Basis von Camelina-Öl und/oder Mandelöl eine deutlich höhere Menge Lösungsvermittler benötigen, um eine stabile Einarbeitung des Öls in das Shampoo zu gewährleisten. Beispeielsweise ließ sich das erfindungsgemäße Amaranthsamenöl bei einer bestimmten Konzentration an Lösungsvermittler stabil und klar in ein Shampoo einarbeiten, während eine identische Zusammensetzung mit Camelina-Öl trüb war, und eine mit Mandelöl eine Phasenseparation aufwies.

Ein erster Gegenstand der vorliegenden Erfindung sind daher kosmetische Zubereitungen, die in einem kosmetisch akzeptablen Medium ein aus den Samen der Amaranthpflanze durch Extraktions- oder Pressverfahren gewonnenes Öl und mindestens ein kationisches Pflegepolymer enthalten.

Die Lipidfraktion der Amaranthsamen besteht im wesentlichen aus Triglyceriden. Die Linolsäure stellt dabei neben Öl- und Palmitinsäure die Hauptkomponente in der Fettsäureverteilung des Öls dar. Daneben sind aber auch Myristinsäure, Stearinsäure, Vaccensäure, α-Linolensäure, Arachinsäure, 11-Eicosensäure und Behensäure im Fettsäurespektrum vertreten. Besonders hervorzuheben ist allerdings ein besonders hoher Anteil an Squalen (6-12%) in Amaranthsamenöl, das in anderen konventionellen Pflanzenölen nur in sehr geringen Konzentrationen zu finden ist Zudem ist das Amaranthsamenöl reich an zahlreichen Vitamin-E-Derivaten (u.a. α-, β-, γ- und δ-Tocopherol sowie α- und β-Tocotrienol) und weist einen bemerkenswerten Anteil an den Phytosterolen Δ7-Avenasterol, Δ7-Campesterol, Δ7-Sitosterol und Δ7-Stigmasterol auf. Ein erfindungsgemäß geeignetes Öl ist beispielsweise unter der Handelsbezeichnung "Amaranth Seed Oil" von der Fa. Euro Ingredients erhältlich.

In den kosmetischen Zusammensetzungen ist das zuvor beschriebene Amaranthsamenöl - bezogen auf ihr Gesamtgewicht - in einer Menge von 0,01 bis 5 Gew.-%, bevorzugt in einer Menge von 0,02 bis 4 Gew.-%, und insbesondere in einer Menge von 0,05 bis 2 Gew.-% enthalten.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (I), 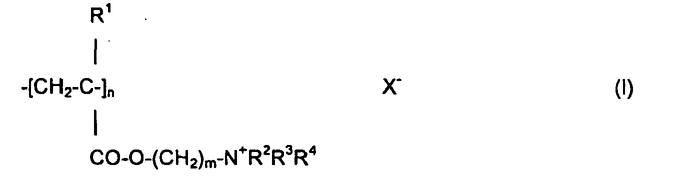 in der R¹= -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.
Weitere bevorzugte kationische Polymere sind beispielsweise
- quatemisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind. Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quatemisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein. Ganz besonders bevorzugt sind die kationischen Proteinhydrolysate und -derivate auf pflanzlicher Basis.

Erfindungsgemäß besonders bevorzugte kationische Polymere sind kationische Cellulose-Derivate sowie Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Die kationischen Polymere werden in den erfindungsgemäßen Zusammensetzungen - bezogen auf ihr Gesamtgewicht - in einer Menge von 0,01 bis 5 Gew.-%, bevorzugt in einer Menge von 0,1 bis 4 Gew.-%, und insbesondere in einer Menge von 0,2 bis 3 Gew.-% eingesetzt In einer zweiten bevorzugten Ausführungsform enthalten die erfindungsgemäßen Zusammensetzungen weiterhin
i) mindestens ein Vitamin und/oder mindestens eine Vitaminvorstufe und/oder ein Derivat der Vitamine und/oder Vitaminvorstufe und/oder
ii) mindestens einen Pflanzenextrakt und/oder
iii) mindestens ein Protein und/oder mindestens ein Proteinhydrolysat und/oder
iv) mindestens eine Silikonverbindung.

Erfindungsgemäß bevorzugte Vitamine, Pro-Vitamine und Vitaminvorstufen sind solche, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%. bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten. Bevorzugt enthalten die erfindungsgemäß verwendeten Mittel Vitamine, Provitamine und Vitaminvorstufen aus den Gruppen A, B, E und H.

Panthenol, Pantolacton, Pyridoxin und seine Derivate sowie Nicotinsäureamid und Biotin sind besonders bevorzugt.

Erfindungsgemäß geeignete Pflanzenextrakte werden üblicherweise durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Erfindungsgemäß geeignete Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Selbstverständlich umfaßt die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in den Mitteln in Konzentrationen von 0,01 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Unter dem Begriff Silikonöle versteht der Fachmann mehrere Strukturen siliciumorganischer Verbindungen. Zunächst werden hierunter die Dimethiconole (S1) verstanden. Dimethiconole bilden die erste Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (S1 - I) dargestellt werden:

(SiOHR¹₂)-O-(SiR²₂-O-)ₓ-(SiOHR¹₂) (S1-I)

Verzweigte Dimethiconole können durch die Strukturformel (S1 - II) dargestellt werden: 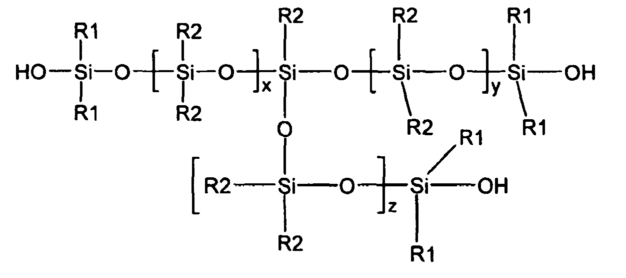 Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS ( beide Guardian Laboratories), Nonychosine E, Nonychosine V (beide Exsymol), SanSurf Petrolatum-25, Satin Finish ( beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Wenn die Dimethiconole (S1) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Dimethiconol bezogen auf die Zusammensetzung.

Dimethicone (S2) bilden die zweite Gruppe der Silikone, welche erfindungsgemäß besonders bevorzugt sind. Die erfindungsgemäßen Dimethicone können sowohl linear als auch verzweigt als auch cyclisch oder cyclisch und verzweigt sein. Lineare Dimethicone können durch die folgende Strukturformel (S2 -I) dargestellt werden:

(SiR¹₃)-O-(SiR²₂-O-)ₓ-(SiR¹₃) (S2 - I)

Verzweigte Dimethicone können durch die Strukturformel (S2 - II) dargestellt werden: 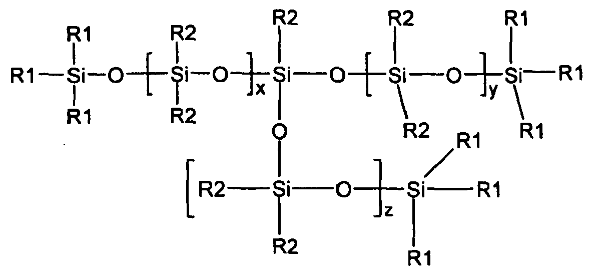 Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs. Der bevorzugteste Bereich liegt zwischen 50000 und 2000000 cPs.

Wenn die Dimethicone (S2) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Dimethiconol bezogen auf die Zusammensetzung.

Dimethiconcopolyole (S3) bilden eine weitere Gruppe bevorzugter Silikone. Dimethiconole können durch die folgende Strukturformeln dargestellt werden:

(SiR¹₃)-O-(SiR²₂-O)ₓ-(SiRPE-O-)_{y}-(SiR¹₃) (S3 - I)

oder durch die nachfolgende Strukturformel:

PE-(SiR¹₂)-O-(SiR²₂-O-)ₓ-(SiR¹₂)-PE (S3 - II)

Verzweigte Dimethiconcopolyole können durch die Strukturformel (S3 - III) dargestellt werden: 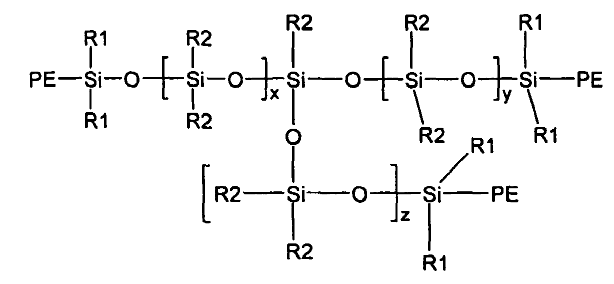 oder durch die Strukturformel(S3 - IV): 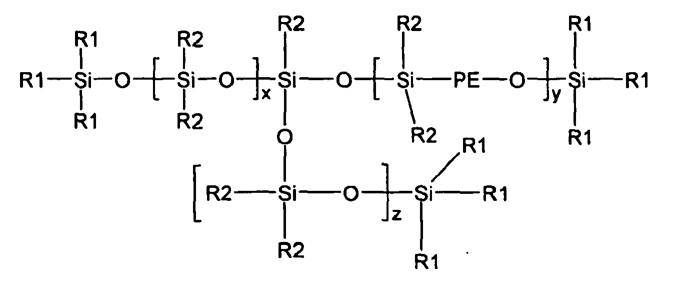 Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein. Bevorzugt als R¹ und R² sind Methyl, Phenyl und C2 bis C22 - Alkylreste.

Bei den C2 bis C22 Alkylresten sind ganz besonders Lauryl-, Stearyl-, und Behenylreste bevorzugt. PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25°C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs.
Der bevorzugteste Bereich liegt zwischen 50000 und 2000000 cPs.

Wenn die Dimethiconcopolyole (S3) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Dimethiconcopolyol bezogen auf die Zusammensetzung.

Aminofunktionelle Silikone oder auch Amodimethicone (S4) genannt, sind Silicone, welche mindestens eine (gegebenenfalls substituierte) Aminogruppe aufweisen.

Solche Silicone können z.B. durch die Formel (S4 - I)

M(RₐO_{b}SiO_{(4-a-b)/2)x}(R_{c}SiO_{(4-c)/2)y}M (S4 - I)

Beschreiben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, - CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H₄C₆H₄-, -C₆H ₄CH₂C₆H₄-; und -(CH ₂)₃C(O)SCH₂CH₂- ein.
Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und z 0 ist.
Q ist am bevorzugtesten ein polarer, aminfunktioneller Rest der Formel-CH₂CH₂CH₂NHCH₂CH₂NH₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.
Bevorzugte erfindungsgemäße Mittel sind dadurch gekennzeichnet, daß sie ein aminofunktionelles Silikon der Formel (S4 - 11)

R'ₐG₃₋ₐ-Si(OSiG₂)ₙ-(OSiG_{b}R'_{2-b})ₘ-O-SiG₃₋ₐ-R'ₐ (S4 -II).

enthalten, worin bedeutet:
- G ist-H, eine Phenylgruppe, -OH, -O-CH₃, -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   o -N(R")-CH₂-CH₂- N(R")₂
   o -N(R")₂
   o -N⁺(R")₃A⁻
   o -N⁺H(R")₂ A⁻
   o -N⁺H₂(R")A⁻
   o -N(R")-CH₂-CH₂-N⁺R"H₂A⁻,
      wobei jedes R" für gleiche oder verschiedene Reste aus der Gruppe -H, - Phenyl, -Benzyl, der C₁₋₂₀-Alkylreste, vorzugsweise -CH₃, -CH₂CH₃, - CH₂CH₂CH₃, -CH(CH₃)₂, -CH₂CH₂CH₂H₃, -CH₂CH(CH₃)₂, -CH(CH₃)CH₂CH₃, - C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, daß sie ein aminofunktionelles Silikon der Formel (S4 - III) 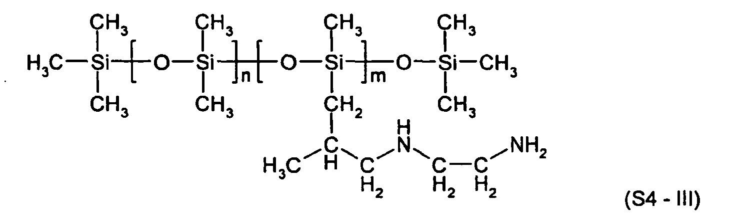 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt. Diese Silicone werden nach der INCI-Deklaration als Trimethytsilylamodimethicone bezeichnet. Besonders bevorzugt sind auch erfindungsgemäße Mittel, die dadurch gekennzeichnet sind, daß sie ein aminofunktionelles Silikon der Formel (S4 - IV) 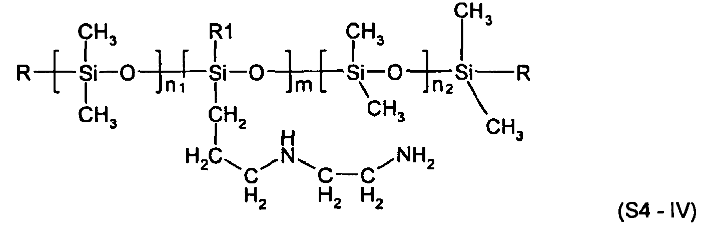 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, bei denen das aminofunktionelle Silikon eine Aminzahl oberhalb von 0,25 meq/g, vorzugsweise oberhalb von 0,3 meq/g und insbesondere oberhalb von 0,4 meq/g aufweist. Die Aminzahl steht dabei für die Milli-Äquivalente Amin pro Gramm des aminofunktioinelen Silicons. Sie kann durch Titration ermittelt und auch in der Einheit mg KOH/g angegeben werden.

Wenn die Amodimethicone (S4) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Amodimethicon bezogen auf die Zusammensetzung.

Die Erfindung umfasst selbstverständlich auch die Erkenntnis, dass in den erfindungsgemäßen Zusammensetzungen (1 und 2) eine Mischung aus mindestens 2 unterschiedlichen Silikonen verwendet werden kann. Bevorzugte Mischungen verschiedener Silikone sind beispielsweise Dimethicone und Dimethiconole, lineare Dimethicone und cylische Dimethiconole. Eine ganz besonders bevorzugte Mischung von Silikonen besteht aus mindestens einem cyclischen Dimethiconol und/oder Dimethicon, mindestens einem weiteren nicht cylischen Dimethicon und/oder Dimethiconol sowie mindestens einem aminofunktionellem Silikon. Werden unterschiedliche Silikone als Mischung verwendet, so ist das Mischungsverhältnis weitgehend variabel. Bevorzugt werden jedoch alle zur Mischung verwendeten Silikone in einem Verhältnis von 5 : 1 bis 1 : 5 im Falle einer binären Mischung verwendet. Ein Verhältnis von 3 : 1 bis 1 : 3 ist besonders bevorzugt. Ganz besonders bevorzugte Mischungen enthalten alle in der Mischung enthaltenen Silikone weitestgehend in einem Verhältnis von etwa 1 : 1, jeweils bezogen auf die eingesetzten Mengen in Gew.%.

Wenn die Silikon mischung in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Silikonmischung bezogen auf die Zusammensetzung.

In einer dritten bevorzugten Ausführungsform der Erfindung enthalten die erfindungsgemäßen Zubereitungen weiterhin mindestens ein Tensid.

Als anionische Tenside (E1) eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 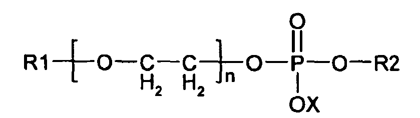 in der R¹ bevorzugt für einen aliphatischen Kohlenwasserstoffrest mit 8 bis 30 Kohlenstoffatomen, R² für Wasserstoff, einen Rest (CH₂CH₂O)ₙR oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (E1-III) 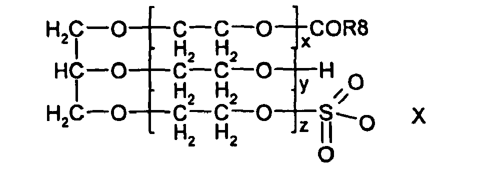 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, wie sie beispielsweise in der EP-B1 0 561 825, der EP-B1-0 561 999, der DE-A1 42 04 700 oder von A.K.Biswas et al. in J.Am.Oil.Chem.Soc. 37, 171 (1960) und F.U.Ahmed in J.Am.OiLChem.Soc. 67, 8 (1990) beschrieben worden sind,
- Amidethercarbonsäuren wie sie in der EP 0 690 044 beschrieben sind,
- Kondensationsprodukte aus C₈ - C₃₀ - Fettalkoholen mit Proteinhydrolysaten und/oder Aminosäuren und deren Derivaten,welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder - SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat. Unter ampholytischen Tensiden (E3) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂- C₁₈ - Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₈ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol -Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen
   Zuckerrest mit 5 oder 6 Kohlenstoffatomen und p für Zahlen von 1 bis 10 steht.
- Zuckertenside vom Typ der Fettsäure-N-alkylpolyhydroxyalkylamide, ein nichtionisches Tensid der Formel (E4-III),

   R⁵CO-NR⁶-[Z] (E4-III)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.%.

Die Tenside werden in Mengen von 0,1 - 45 Gew.%, bevorzugt 0,5 - 30 Gew.% und ganz besonders bevorzugt von 0,5 - 25 Gew.%, bezogen auf das gesamte erfindungsgemäß verwendete Mittel, eingesetzt.

Erfindungsgemäß einsetzbar sind ebenfalls kationische Tenside (E5) vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Oehyquart^{®} F-75, Dehyquart^{®}C-4046, Dehyquart^{®} L80 und Dehyquart^{®} AU-35 sind Beispiele für solche Esterquats. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside (E5) sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Anionische, nichtionische, zwitterionische und/oder amphotere Tenside sowie deren Mischungen können erfindungsgemäß bevorzugt sein.

In einer weiteren bevorzugten Ausführungsform der Erfindung kann die Wirkung der erfindungsgemäßen Zubereitungen durch Fettstoffe weiter gesteigert werden. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren können eingesetzt werden lineare und/oder verzweigte, gesättigte und/oder ungesättigte Fettsäuren mit 6-30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäute, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1-5 Gew.% sein können.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureester erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} 0 oder Lorof^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, lsofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 -15 Gew.% bezogen auf das gesamte Mittel.

Zu den natürlichen und synthetischen kosmetischen Ölkörpern, welche die Wirkung der erfindungsgemäßen Zubereitungen steigern können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugte sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/- caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I), 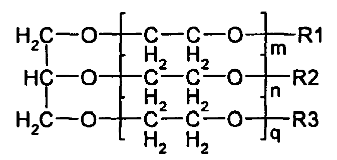 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, daß mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Olkörper in den erfindungsgemäßen Mitteln beträgt üblicherweise 0,1 - 30 Gew.%, bezogen auf das gesamte Mittel, bevorzugt 0,1 - 20 Gew.-%, und insbesondere 0,1 -15 Gew.-%.

Die Gesamtmenge an Öl- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Als weiterhin vorteilhaft hat es sich gezeigt, dass weitere Polymere die Wirkung der erfindungsgemäßen Zubereitungen weiter unterstützen können. In einer bevorzugten Ausführungsform werden den erfindungsgemäßen Mitteln daher Polymere zugesetzt, wobei sich sowohl anionische, amphotere als auch nichtionische Polymere als wirksam erwiesen haben.

Bei den anionischen Polymeren, welche die Wirkung der erfindungsgemäßen Zubereitungen unterstützen können, handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvemetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze^{®} QM im Handel erhältlich.

Weiterhin können als Polymere zur Steigerung der Wirkung der erfindungsgemäßen Zubereitungen amphotere Polymere verwendet werden. Unter dem Begriff amphotere Polymere werden sowohl solche Polymere, die im Molekül sowohl freie Aminogruppen als auch freie - COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylamincethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N(⁺)R³R⁴R⁵ A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3-II),

   R⁶-CH=CR⁷-COOH (G3-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Mittel können in einer weiteren Ausführungsform nichtionogene Polymere enthalten.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils VinylpyrrolidonNinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200°C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone gemäß der EP 0612759 B1.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Die Polymere sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Weiterhin kann in einer bevorzugten Ausführungsform der Erfindung die Wirkung der Zubereitungen durch UV - Filter gesteigert werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestem, Diphenylacrylsäureestem, Zimtsäureestern, Salicylsäureestern, Benzimidazolen und o-Aminobenzoesäureestern.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalo^{®} HP 610). Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt. Die Wirkung der erfindungsgemäßen Zubereitungen kann weiterhin durch eine 2-Pyrrolidinon-5-carbonsäure und deren Derivate gesteigert werden. Ein weiterer Gegenstand der Erfindung ist daher die Verwendung des Wirkstoffes in Kombination mit Derivaten der 2-Pyrrolidinon-5-carbonsäure. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen tragt Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Zusätzlich kann es sich als vorteilhaft erweisen, wenn die erfindungsgemäßen Zubereitungen Penetrationshilfsstoffe und/oder Quellmittel enthalten. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Vorteilhaft im Sinne der Erfindung können zusätzlich kurzkettige Carbonsäuren die Zubereitungen unterstützen. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Als Beispiele für erfindungsgemäße Carbonsäuren seien genannt Ameisensäure, Essigsäure, Propionsäure, Buttersäure, Isobuttersäure, Valeriansäure, Isovaleriansäure, - Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxyphthalamidsäure, 1-Pyrazolcarbonsäure, Gallussäure oder Propantricarbonsäure.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren gemeinsam mit dem Wirkstoff (A) einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8-22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Die erfindungsgemäßen Zubereitungen können auch als Färbemittel, Wellmittel oder Fixiermittel konfektioniert sein.

Der Begriff Färbevorgang umfaßt dabei alle dem Fachmann bekannten Verfahren, bei denen auf das, gegebenenfalls angefeuchtete, Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. K. H. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Der Begriff Wellvorgang umfaßt dabei alle dem Fachmann bekannten Verfahren, bei denen auf das, gegebenenfalls angefeuchtete, und auf Wickler gedrehte Haar ein Wellmittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird, anschließend auf das Haar eine Dauerwellfixierung aufgebracht wird und diese für eine Zeit zwischen wenigen Minuten und ca. 45 Minuten auf dem Haar belassen und anschließend mit Wasser oder einem tensidhaltigen Mittel ausgespült wird. Es wird in diesem Zusammenhang ausdrücklich auf die bekannten Monographien, z. B. K. H. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Hinsichtlich der Art, gemäß welcher der erfindungsgemäße Wirkstoffkomplex auf die keratinische Faser, insbesondere das menschliche Haar, aufgebracht wird, bestehen keine prinzipiellen Einschränkungen. Als Konfektionierung dieser Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Gele, Sprays, Aerosole und Schaumaerosole geeignet. Der pH-Wert dieser Zubereitungen kann prinzipiell bei Werten von 2 - 11 liegen. Er liegt bevorzugt zwischen 3 und 11, wobei Werte von 4 bis 10 besonders bevorzugt sind. Zur Einstellung dieses pH-Wertes kann praktisch jede für kosmetische Zwecke verwendbare Säure oder Base verwendet werden. Bevorzugte Basen sind Ammoniak, Alkalihydroxide, Monoethanolamin, Triethanolamin sowie N,N,N',N'-Tetrakis-(2-hydroxypropyl)-ethylendiamin.

Auf dem Haar verbleibende Zubereitungen haben sich als wirksam erwiesen und können daher bevorzugte Ausführungsformen der erfindungsgemäßen Lehre darstellen. Unter auf dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wäßrigen Lösung wieder aus dem Haar ausgespült werden. Vielmehr verbleiben die Zubereitungen bis zur nächsten Haarwäsche, d.h. in der Regel mehr als 12 Stunden, auf dem Haar.

Gemäß einer zweiten bevorzugten Ausführungsform werden diese Zubereitungen als Haarkur oder Haar-Conditioner formuliert. Die erfindungsgemäßen Zubereitungen gemäß dieser Ausführungsform können nach Ablauf dieser Einwirkzeit mit Wasser oder einem zumindest überwiegend wasserhaltigen Mittel ausgespült werden; sie können jedoch, wie oben ausgeführt, auf dem Haar belassen werden. Dabei kann es bevorzugt sein, die erfindungsgemäße Zubereitung vor der Anwendung eines reinigenden Mittels, eines Wellmittels oder anderen Haarbehandlungsmitteln auf das Haar aufzubringen. In diesem Falle dient die erfindungsgemäße Zubereitung als Strukturschutz für die nachfolgenden Anwendungen.

Gemäß weiteren bevorzugten Ausführungsformen kann es sich bei den erfindungsgemäßen Mitteln aber beispielsweise auch um reinigende Mittel wie Shampoos, pflegende Mittel wie Spülungen, festigende Mittel wie Haarfestiger, Schaumfestiger, Styling Gels und Fönwellen, dauerhafte Verformungsmittel wie Dauerwell- und Fixiermittel sowie insbesondere im Rahmen eines Dauerwellverfahrens oder Färbeverfahrens eingesetzte Vorbehandlungsmittel oder Nachspülungen handeln.

Neben den vorgenannten, erfindungsgemäß zwingend erforderlichen und fakultativen Inhaltsstoffen können die Zubereitungen dieser Erfindung prinzipiell alle weiteren, dem Fachmann für solche kosmetischen Mittel bekannten Komponenten enthalten.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Parfümöle, Dimethylisosorbid und Cyclodextrine,
- faserstrukturverbessemde Wirkstoffe, insbesondere Mono-, Di- und Oligosaccharide, wie beispielsweise Glucose, Galactose, Fructose, Fruchtzucker und Lactose,
- Farbstoffe zum Anfärben des Mittels,
- Antischuppenwirkstoffe wie Piroctone Olamine, Zink Omadine und Climbazol,
- Wirkstoffe wie Allantoin und Bisabolol,
- Cholesterin,
- Komplexbildner wie EDTA, NTA, β-Alanindiessigsäure und Phosphonsäuren,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und α-Mercaptoethansulfonsäure,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die oben genannte Monographie von K. H. Schrader verwiesen.

Für den Fall, dass die erfindungsgemäßen Zubereitungen als Aufhell-, Färbe- oder Tönungsmittel konfektioniert werden, enthalten sie im Falle der Färbe- und Tönungsmittel neben den vorstehend genannten Inhaltsstoffen weiterhin Farbstoffe und/oder Farbstoffvorprodukte. Dabei können die erfindungsgemäßen Mittel direktziehende Farbstoffe, Oxidationsfarbstoffvorprodukte vom Entwickler- und/oder Kupplertyp sowie Vorstufen naturanaloger Farbstoffe enthalten.

Ein weiterer Gegenstand der Erfindung ist Verwendung der erfindungsgemäßen Zubereitungen zur Reinigung und/oder Pflege der Haare.

Gegenstand der Erfindung ist auch die Verwendung erfindungsgemäßen Zubereitungen zur Verbesserung der Kämmeigenschaften und zur Erhöhung des Glanzes keratinischer Fasern, insbesondere menschlicher Haare.

Gegenstand der Erfindung ist auch ein Verfahren zur Haarbehandlung, bei dem eine erfindungsgemäße Zubereitung auf die Fasern aufgetragen wird, und nach einer Einwirkzeit von 1 bis 45 Minuten wieder ausgespült wird.

### Beispiele

Alle Mengenangaben sind, soweit nicht anders vermerkt, Gewichtsteile.

### Anwendungsbeispiele

| **Haarshampoo** | |
|---|---|
| Citronensäure | 0,5 |
| Na-Laurethsulfat 25% | 50 |
| Disodium Cocoamphodiacetate | 7 |
| Salicylsäure | 0,2 |
| D-Panthenol 75% | 0,5 |
| Na-benzoat | 0,5 |
| Euperlan^{®} PK 3000 AM¹ | 2 |
| Cetiol^{®} HE² | 1 |
| Amaranth Öl³ | 0,2 |
| Polymer JR^{®}400⁴ | 0,5 |
| Parfüm | 0,5 |
| PEG-40 Hydrogenated Castor Oil 455 | 1 |
| Macadamianussöl, raffiniert | 0,2 |
| NaCl | 0,5 |
| Wasser | ad 100 |

| **Haar-Aufbaucreme** | |
|---|---|
| Paraffinum Liquidum | 1 |
| Dehyquart^{®} F 75⁵ | 2 |
| Amaranth Öl | 1 |
| Varisoft^{®} W 75 PG⁶ | 1,5 |
| Cetearyl Alcohol | 3,5 |
| Emulmetik^{®} 100⁷ | 0,4 |
| Propylparaben | 0,15 |
| Cutina^{®} CP⁸ | 0,7 |
| Stearamidopropyldimethylamine | 1 |
| Dehyquart^{®} A CA⁹ | 3 |
| Citronensäure | 0,5 |
| Methylparaben | 0,15 |
| Phenoxyethanol | 0,4 |
| D-Panthenol 75% | 0,2 |
| Gluadin^{®} W 20¹⁰ | 1 |
| Parfüm | 0,4 |
| Salcare^{®} SC 96" | 1 |
| Wasser | ad 100 |

| **Leave-In-Treatment** | |
|---|---|
| Synthalen^{®} K¹² | 0,25 |
| Sepigel^{®} 305¹³ | 2,6 |
| Dehyquart^{®} F 75⁵ | 0,5 |
| Polymer^{®} JR 400⁴ | 0,3 |
| Neutrol^{®} TE¹⁴ | 0,4 |
| Amaranth Öl | 0,75 |
| Parfüm | 0,3 |
| D-Panthenol 75% | 0,5 |
| Luviskol^{®} K 30 Pulver¹⁵ | 0,5 |
| Gafquat^{®} 755N¹⁶ | 0,5 |
| Dow Corning^{®} 1401 Fluid¹⁷ | 1 |
| Ethanol 96% DEP vergällt | 15 |
| Wasser | ad 100 |

| | |
|---|---|
| ¹ C12 - C16 Fettalkoholglycosid ca. 50% Aktivsubstanz (INCI - Bezeichnung: Lauryl Glucoside) (COGNIS) ² Polyol-Fettsäure-Ester (INCI - Bezeichnung: PEG-7 Glyceryl Cocoate) (COGNIS) ³ Extrakt aus Amarathsamen (EURO-INGREDIENTS) ⁴ quaternierte Hydroxyethylcellulose (INCI - Bezeichnung: Polyquaternium-10) (UNION CARBIDE) ⁵ Fettalkohole-Methyltriethanolammoniummethylsulfatdialkylester-Gemisch (INCI-Bezeichnung: Distearoylethyl Hydroxyethylmonium Methosulfate, Cetearyl Alcohol) (COGNIS) ⁶ 1-Methyl-2-nortalgalkyl-3-talgfettsäureamidoethylimidazolinium-methosulfat in Propylenglycol ca. 74-77% Aktivsubstanz (INCI-Bezeichnung: Quaternium-27, Propylene Glycol) (GOLDSCHMIDT-REWO) ⁷ Phospholipide mit gesättigtem Öl als Träger (INCI-Bezeichnung: Lecithin) (DEGUSSA) ⁸ Cetylpalmitat pflanzlich (INCI-Bezeichnung: Cetyl palmitate) (COGNIS) ⁹ Trimethylhexadecylammoniumchlorid ca. 25% Aktivsubstanz (INCI-Bezeichnung: Cetrimonium Chloride) (COGNIS) ¹⁰ Weizenproteinhydrolysat (20 % Aktivsubstanz in Wasser; INCI-Bezeichnung: Aqua (and) Hydrolized Wheat Protein (and) Sodium Benzoate (and) Phenoxyethanol (and) Methylparaben (and) Propylparaben) (COGNIS) 11 N,N,N-Trimethyl-2[(methyl-1-oxo-2-propenyl)oxy]-Ethanaminiumchlorid-Homopolymer (50 % Aktivsubstanz; INCI-Bezeichnung: Polyquaternium-37 (and) Propylenglycol Dicaprilate Dicaprate (and) PPG-1 Trideceth-6) (ALLIED COLLOIDS) ¹² Polyacrylsäure (INCI-Bezeichnung: Carbomer) (3V SIGMA) ¹³ Copolymer aus Acrylamid und 2-Acrylamido-2-methylpropansulfonsäure (INCI-Bezeichnung: Polyacrylamide (and) C₁₃-C₁₄ Isoparaffin (and) Laureth-7) (SEPPIC) ¹⁴ N,N,N,N-Tetrakis-(2-hydroxypropyl)-ethylendiamin-edetol (INCI-Bezeichnung: Tetrahydroxypropyl Ethylenediamine) (BASF) ¹⁵ Polyvinylpyrrodon (INCI-Bezeichnung: PVP) (BASF) ¹⁶ Vinylpyrrolidon Dimethylaminoethylmethacrylest Copolymer Diethylsulfat quaternisiert, (INCI-Bezeichnung: Polyquaternium-11) (ISP) ¹⁷ Dimethylcyclosiloxan, Dimethylplysiloxanol Gemisch (INCI-Bezeichnung: Cyclomethicone, Dimethiconol) DOW CORNING | |

| **Intensiv-Kur** | |
|---|---|
| Varisoft^{®} W 75 PG⁶ | 1,5 |
| Paraffinum Liquidum | 1 |
| Dehyquart^{®} F 75⁵ | 2 |
| Stearamidopropyldimethylamine | 1 |
| Amaranth Öl | 1,5 |
| Cetearyl Alcohol | 5 |
| Cutina^{®} GMS-V¹⁸ | 1 |
| Citronensäure | 0,3 |
| Dehyquart^{®} A CA⁹ | 5 |
| Salcare^{®} SC 96" | 0,5 |
| Parfüm | 0,4 |
| D-Panthenol 75% | 0,5 |
| Gluadin^{®} W 20¹⁰ | 1 |
| Wasser | ad 100 |

| | |
|---|---|
| ¹⁸ Glycerinmono/dipalmitat/stearat (INCI-Bezeichnung: Glyceryl Stearate) (COGNIS) | |

**Haarfärbemittel**

| **1. Färbecreme, Nuance Hellblond** | |
|---|---|
| Stenol 1618¹⁹ | 6,00 |
| Kokosfettalkohol | 2,00 |
| Eumulgin B1²⁴ | 0,50 |
| Eumulgin B2²⁵ | 0,50 |
| Amaranthöl | 1,50 |
| Akyposott 45NV²⁷ | 10,00 |
| Texapon K14 S70C²⁸ | 2,80 |
| Lamesoft PO65²⁹ | 1,50 |
| Luviquat FC 550³⁰ | 1,50 |
| Ammoniumsulfat | 0,50 |
| Natriumsulfit | 0,30 |
| Ascorbinsäure | 0,20 |
| EDTA-diNa-Salz | 0,30 |
| Parfümöl | 0,30 |
| p-Toluylendiaminsulfat | 0,30 |
| Resorcin | 0,03 |
| 2-Methylresorcin | 0,14 |
| Wasser | ad 100,00 mit Ammoniak auf pH 10 |

| | |
|---|---|
| ²⁷ C₁₂₋₁₄-Fettalkohole Essigsäure Natriumsalz (+4,5 EO) (INCI-Bezeichnung: Sodium Laureth-Carboxylate) (21% Aktivsubstanz) (KAO) ²⁸ Sodium lauryl myristyl ehter Sulfate (+3EO) (INCI-Bezeichnung: Sodium laureth Sulfate) (68-73 % Aktivsubstanz) (COGNIS) ²⁹ Alkylpolyglucosid Ölsäuremonoglycerid-Gemisch (INCI-Bezeichnung: Coco-glucoside, Glyceryl Oleate, AQUA) (FK: 65-70%) (COGNIS) ³⁰ 3-Methylvinylimidazolinchlord Vinylpyrrolidon Copolymerisat (INCI-Bezeichnung: Polyquaternium-16) (FLK: 38-42%) (BASF) | |

| **2. Entwicklerdispersion** | |
|---|---|
| Wasserstoffperoxid 50%-ig | 12,00 |
| Turpinal SL²¹ | 1,50 |
| Texapon NSO²⁰ | 2,00 |
| Aculyn 33³¹ | 12,00 |
| Wasser | ad 100,00 |
| Beide Komponenten werden vor der Anwendung | im Verhältnis 1:1 gemischt. |

| | |
|---|---|
| ³¹ INCI-Bezeichnung: Acrylates Copolymer (ISP) | |

### Wirkungsnachweis:

Es wurden erfindungsgemäße Shampoos mit Amaranthsamenöl und nicht erfindungsgemäße Shampoos miteinander verglichen, und hinsichtlich den Eigenschaften
- Glanz
- Griff, trocken
- Kämmbarkeit, trocken und
- Griff, nass
   auf ungeschädigtem Haar sensorisch bewertet.

Die Beurteilung erfolgte durch vier Testpersonen in einer Blindprobe, auf einer Skala von 1-5 (1= sehr schlecht, 2= schlecht, 3=mittelmäßig, 4= gut, 5= sehr gut). Aus den einzelnen Beurteilungen wurden die Mittelwerte gebildet.

| | Shampoos mit 0,05% Amaranthöl | Shampoos mit 0,3% Polyquaternium-10 |
|---|---|---|
| Glanz | 3,75 | 3,25 |
| Griff, trocken | 4,00 | 3,25 |

| | Shampoos mit 0,3% Polyquaternium-10 und 0,5% Amaranthöl | Shampoos mit 0,3% Polyquaternium-10 und 0,5% Mandelöl |
|---|---|---|
| Glanz | 4,00 | 3,60 |
| Griff, trocken | 4,25 | 3,80 |
| Kämmbarkeit, trocken | 4,25 | 3,60 |
| Griff, nass | 3,67 | 3,25 |

Die Versuche zeigen eindeutig, dass ein Gehalt von Amaranthöl in Shampooformulierungen vorteilhaft gegenüber vergleichbaren Rezepturen aus dem Stand der Technik sind.

## Patentansprüche

1. Kosmetische Zubereitungen zur Haarbehandlung, enthaltend in einem kosmetisch akzeptablen Medium
a) ein aus den Samen der Amaranthpflanze durch Extraktion- oder Pressverfahren gewonnenes Öl und
b) mindestens ein kationisches Pflegepolymer

2. Kosmetische Zubereitungen nach Anspruch 1, **dadurch gekennzeichnet, dass** sie das Amaranthsamenöl - bezogen auf ihr Gesamtgewicht - in einer Menge von 0,01 bis 5 Gew.-%, bevorzugt in einer Menge von 0,02 bis 4 Gew.-% und insbesondere in einer Menge von 0,05 bis 2 Gew.-% enthalten.

3. Kosmetische Zubereitungen nach einem der Ansprüche 1 oder 2, **dadurch gekennzeichnet, dass** sie weiterhin
i) mindestens ein Vitamin und/oder mindestens eine Vitaminvorstufe und/oder ein Derivat der Vitamine und/oder Vitaminvorstufe und/oder
ii) mindestens einen Pflanzenextrakt und/oder
iii) mindestens ein Protein und/oder mindestens ein Proteinhydrolysat und/oder
iv) mindestens eine Silikonverbindung
enthalten.

4. Kosmetische Zubereitungen nach einem der Ansprüche 1 bis 3, **dadurch gekennzeichnet, dass** sie weiterhin Verbindungen aus der Gruppe der Tenside enthalten.

5. Kosmetische Zubereitungen nach einem der Ansprüche 1 bis 4, **dadurch gekennzeichnet, daß** sie zusätzlich mindestens einen direktziehenden Farbstoff enthalten.

6. Kosmetische Zubereitungen nach einem der Ansprüche 1 bis 5, **dadurch gekennzeichnet, daß** sie mindestens eine Entwicklerkomponente und gegebenenfalls zusätzlich mindestens eine Kupplerkomponente enthalten.

7. Kosmetische Zubereitungen nach einem der Ansprüche 1 bis 6 **dadurch gekennzeichnet, daß** sie zusätzlich mindestens ein Indol- und /oder Indolinderivat als Vorprodukt eines naturanalogen Farbstoffs enthalten.

8. Kosmetische Zubereitungen nach einem der Ansprüche 1 bis 7, **dadurch gekennzeichnet, daß** sie zusätzlich mindestens ein Oxidationsmittel, insbesondere Wasserstoffperoxid, enthalten.

9. Kosmetische Zubereitungen nach Anspruch 8, **dadurch gekennzeichnet, daß** sie zusätzlich mindestens einen Bleichverstärker enthalten.

10. Verwendung einer Zubereitung nach einem der Ansprüche 1 bis 9 zur Reinigung und/oder Pflege der Haare.

11. Verwendung einer Zubereitung nach einem der Ansprüche 1 bis 9 zur Verbesserung der Kämmeigenschaften und zur Erhöhung des Glanzes keratinischer Fasern, insbesondere menschlicher Haare.

12. Verfahren zur Haarbehandlung, bei dem eine Zubereitung gemäß einem der Ansprüche 1 bis 9 auf die Fasern aufgetragen wird, wobei die Zubereitung nach einer Einwirkzeit von 1 bis 45 Minuten wieder ausgespült wird.

## Claims

1. Cosmetic preparations for hair treatment, containing in a cosmetically acceptable medium
a) an oil obtained from the seed of the amaranth plant by an extraction or pressing method and
b) at least one cationic conditioning polymer.

2. The cosmetic preparations according to claim 1, **characterized in that** they contain the amaranth seed oil in an amount from 0.01-5% by weight, preferably in an amount from 0.02-4% by weight, and in particular in an amount from 0.05-2% by weight, based on their total weight.

3. The cosmetic preparations according to claims 1 or 2, **characterized in that** they further contain
i) at least one vitamin and/or at least one vitamin precursor and/or a derivative of vitamins and/or vitamin precursor and/or
ii) at least one plant extract and/or
iii) at least one protein and/or at least one protein hydrolyzate and/or
iv) at least one silicone compound.

4. The cosmetic preparations according to any of claims 1 to 3, **characterized in that** they further contain compounds from the group of surfactants.

5. The cosmetic preparations according to any of claims 1 to 4, **characterized in that** they additionally contain at least one direct dye.

6. The cosmetic preparations according to any of claims 1 to 5, **characterized in that** they contain at least one developer component and possibly additionally at least one coupling component.

7. The cosmetic preparations according to any of claims 1 to 6, **characterized in that** they contain additionally at least one indole and/or indoline derivative as a precursor product of a nature-analog dye.

8. The cosmetic preparations to any of claims 1 to 7, **characterized in that** they additionally contain at least one oxidation agent, in particular hydrogen peroxide.

9. The cosmetic preparations according to claim 8, **characterized in that** they additionally contain at least one bleach booster.

10. The use of a preparation according to any of claims 1 to 9 for cleaning and/or conditioning hair.

11. The use of a preparation according to any of claims 1 to 9 for improving combing properties and increasing the shine of keratinous fibers, in particular human hair.

12. A method for hair treatment, in which a preparation according to any of claims 1 to 9 is applied on the fibers, wherein the preparation is again rinsed off after a contact time from 1 to 45 minutes.

## Revendications

1. Préparations cosmétiques pour le traitement capillaire, contenant, dans un milieu acceptable du point de vue cosmétique :
a) une huile que l'on obtient à partir des graines de la plante d'amarante via un procédé par extraction ou par pressage, et
b) au moins un polymère cationique de soin.

2. Préparations cosmétiques selon la revendication 1, **caractérisées en ce qu'**elles contiennent l'huile de graine d'amarante - rapporté à son poids total - en une quantité de 0,01 à 5 % en poids, de préférence en une quantité de 0,02 à 4 % en poids et en particulier en une quantité de 0,05 à 2 % en poids.

3. Préparations cosmétiques selon l'une quelconque des revendications 1 ou 2, **caractérisées en ce qu'**elles contiennent en outre
i) au moins une vitamine et/ou au moins un précurseur de vitamine et/ou un dérivé des vitamines et/ou des précurseurs de vitamines et/ou
ii) au moins un extrait de plante et/ou
iii) au moins une protéine et/ou au moins un hydrolysat de protéine et/ou
iv) au moins un composé à base de silicone.

4. Préparations cosmétiques selon l'une quelconque des revendications 1 à 3, **caractérisées en ce qu'**elles contiennent en outre des composés du groupe des agents tensioactifs.

5. Préparations cosmétiques selon l'une quelconque des revendications 1 à 4, **caractérisées en ce qu'**elles contiennent en outre au moins un colorant direct.

6. Préparations cosmétiques selon l'une quelconque des revendications 1 à 5, **caractérisées en ce qu'**elles contiennent au moins un composant faisant office de développeur et le cas échéant en outre au moins un composant faisant office de coupleur.

7. Préparations cosmétiques selon l'une quelconque des revendications 1 à 6, **caractérisées en ce qu'**elles contiennent en outre au moins un dérivé d'indole et/ou un dérivé d'indoline à titre de précurseur d'un colorant analogue à ceux existant dans la nature.

8. Préparations cosmétiques selon l'une quelconque des revendications 1 à 7, **caractérisées en ce qu'**elles contiennent en outre au moins un agent d'oxydation, en particulier du peroxyde d'hydrogène.

9. Préparations cosmétiques selon la revendication 8, **caractérisées en ce qu'**elles contiennent en outre au moins un renforçateur du blanchiment.

10. Utilisation d'une préparation selon l'une quelconque des revendications 1 à 9 pour le lavage et/ou le soin des cheveux.

11. Utilisation d'une préparation selon l'une quelconque des revendications 1 à 9 pour améliorer les propriétés de peignage et pour augmenter le brillant de fibres kératiniques, en particulier de cheveux humains.

12. Procédé pour le traitement capillaire, dans lequel on applique sur les fibres une préparation selon l'une quelconque des revendications 1 à 9, la préparation étant à nouveau éliminée par rinçage après l'avoir laissé agir pendant un laps de temps de 1 à 45 minutes.
